# EUROPEAN PATENT APPLICATION

(11) **EP 4 624 895 A1**
(43) Date of publication of application: **01.10.2025**
(21) Application number: 23896962.0
(22) Date of filing: 04.12.2023
(51) Int. Cl.: G01N 15/10

(54) **DEVICE FOR VISUAL QUANTIFICATION OF MATERIAL PARTICLES IN SOLUTION**

(30) Priority: 02.12.2022 US 202263385796 P; 19.10.2023 US 202318490124
(71) Applicant: City University of Hong Kong, Kowloon, Hong Kong (CN)
(72) Inventor: CHEN, Ting-Hsuan, Hong Kong, Kowloon Tong Hong Kong (CN); HARTANTO, Hogi, Hong Kong, Kowloon Tong Hong Kong (CN); LI, Jiaheng, Hong Kong, Kowloon Tong Hong Kong (CN)
(74) Representative: Monteiro Alves, Inês
(86) International application number: PCT/CN2023/136212
(87) International publication number: WO 2024/114820

(57) **Abstract**

A device, in particular a microfluidic device, for facilitating visual determination of presence and/or quantification of one or more species in a sample solution. The microfluidic device includes an inlet arranged to receive a solution, and a trap in fluid communication with the inlet and arranged to substantially trap one or more species of a sample solution. The sample solution may be the sample solution received at the inlet or a processed solution obtained by processing the sample solution received at the inlet. The trap includes, at least, a fluid channel. The trap is arranged such that the one or more trapped species is visible for presence determination and/or quantification. A method of use in also described herein.

## Description

### TECHNICAL FIELD

This invention relates to a device for facilitating visual determination of presence and/or quantification of a species in a sample solution.

### BACKGROUND

It is known to detect and analyze target chemical species in a sample for disease diagnosis, environmental monitoring and/or analysis, or related health- or environment-related applications. The detection and analysis usually require dedicated equipment, such as spectrometer, fluorescence microscope, thermal cycler, current meter, etc., which can be cumbersome and bulky, and which may require electric power to operate and so may be unsuitable for use in resource limited settings.

Portable sensors and detectors incorporating lateral flow strips or colorimetric assays are known. These sensors and detectors are convenient to use. However, they may provide only qualitative results, i.e., whether there exists the target chemical species. For applications that require quantitative measurement, UV-Vis spectrometer or lateral flow strip reader is still required to analyze the detected signal to determine spectral absorbance to quantify the optical/fluorescence intensity, which could be a complicated process.

Some devices have been devised to address the above needs.

US non-provisional patent application US15/239,926, filed on 18 Aug 2016, discloses kit and method for determining presence or amount of a target nucleic acid sequence in a sample. It teaches a device suitable for use in determining the presence or amount of the target nucleic acid sequence. The device includes an inlet port, a mixing zone, a first collection zone subjected to a magnetic field, a second collection zone with a micro-channel, a capillary pump, and a reservoir. The micro-channel has a neck portion with a diameter smaller than the size of the loaded polystyrene particle. The micro-channel is preferably marked with indicia for direct measurement of the amount of the loaded polystyrene particle being trapped in the microchannel. This US non-provisional patent application, US15/239,926, is hereby incorporated by reference herein in its entirety.

US non-provisional patent application US15/969,877, filed on 3 May 2018, discloses device and method for visual quantification of an amount of target species in a sample solution. The device includes an inlet port, a mixing zone, a magnetic separation zone subjected to a magnetic field, a collection zone with a micro-channel, a capillary pump, and a reservoir. This US non-provisional patent application, US15/969,877, is also hereby incorporated by reference herein in its entirety.

Microfluidic devices are described by, for example, Olanrewaju, et al., Autonomous microfluidic capillaric circuits replicated from 3D-printed molds", Royal Soc. Chem., Lab Chip, vol. 16, pp. 3804-14, 2016; Gao, et al., A simple and rapid method for blood plasma separation driven by capillary force with an application in protein detection, Anal. Methods, vol. 12, pp. 2560-70, 2020; Mielczarek, et al., Microfluidic blood plasma separation for medical diagnostics: is it worth it?, Lab Chip, vol. 16, pp., 3441-48, 2016; Wang, et al., Portable microfluidiv device with thermometer-like display for real-time visual quantitation of Cadmium (II) contamination in drinking water, Analytica Chimica Acta, vol. 1160, 338444, 2021, https://doi.org/10.1016/j.aca.2021.338444; Wu, et al., Cascade-Amplified Microfluidic Particle Accumulation Enabling Quantification of Lead Ions through Visual Inspection, Sens & Actuators: B. Chemical, vol. 324, 128727, https://doi.org/10.1016/j.snb.2020.128727; Jiang, et al., Microfluidic particle accumulation for visual quantification of copper ions, Microchimica Acta, vol. 188, 176, 2021, https://doi.org/10.1007/s00604-021-04822-0; Wu, et al., Visual quantification of silver contamination in fresh water via accumulative length of microparticles in capillary-driven microfluidic devices, Talanta, vol. 235, 122707, 2021, https://doi.org/10.1016/j.talanta.2021.122707; Zhao, et al., Micrfluidic bead trap as a visual bar for quantitative detection of oligonucleotides, Lab Chip, 2017, DOI: 10.1039/c7lc00836h and Wang, et al., Microfluidic Particle Dam for Visual and Quantitative Detection of Lead Ions, ACS. Sens., vol. 5, pp. 19-23, 2020, DOI: 10.1021/acssensors.9b01945.

### SUMMARY OF THE INVENTION

In an aspect of the invention herein relates to a microfluidic device for facilitating visual determination of a species in a sample solution. The microfluidic device includes an inlet arranged to receive a solution; and a trap in fluid communication with the inlet arranged to substantially trap a species of a sample solution. Depending on embodiments, the sample solution may be the solution received at the inlet or a processed solution obtained by processing the solution received at the inlet. The trap comprises a fluid channel and is arranged such that the trapped species is at least partly visible for visual determination of presence and/or quantification. The species may be in the form of particles, e.g., microparticles, nanoparticles, etc. The fluid channel may be transparent or translucent such that the trapped species are visible.

In some embodiments the fluid channel is straight. In some embodiments the fluid channel is curved.

In some embodiments, the microfluidic device further comprises a capillary pump for causing or facilitating movement of the sample solution towards the trap. The capillary pump may be removable, e.g., removably received in a receptacle or space of the microfluidic device.

In some preferred embodiments, the capillary pump is arranged downstream of the trap which traps the species.

In some embodiments, the capillary pump is operable to substantially block passage of the species into the capillary pump, substantially trapping the species in, for example, the trap.

In some embodiments, the capillary pump is disposed immediately downstream of the fluid channel, i.e., without intervening part(s) between them. In one example, the fluid channel may include a first end closer to the inlet and a second end further away from the inlet, and the second end of the fluid channel terminates at the capillary pump. In some embodiments, the capillary pump is disposed immediately downstream of the trap.

In some embodiments, the capillary pump may, for example, include a microstructure-based capillary pump. The capillary pump may, for example, include a porous-material-based capillary pump.

In some embodiments, the capillary pump comprises a porous-material-based capillary pump, and the porous-material-based capillary pump comprises a filter, e.g., a filter paper. Example filter paper material(s) include, e.g., cellulose, cotton linter derived material(s), etc. In some embodiments, the capillary pump or the porous-material-based capillary pump is made of porous material(s) with pore size(s) or an average pore size smaller than a diameter or average diameter of the species.

In some embodiments, the microfluidic device includes a processing arrangement, e.g., arranged between the inlet and the trap, for processing the sample solution received at the inlet and provide a processed solution. The processing arrangement may alter a characteristic (e.g., composition, volume, etc., or any combination thereof) of the sample solution received at the inlet. The processing arrangement may include, e.g., physical means, chemical means, etc., or any combination thereof, to alter the characteristics of the sample solution received at the inlet. Alternatively, the processing arrangement may be located upstream of the inlet.

In some embodiments, the microfluidic device is devoid of a processing arrangement between the inlet and the trap such that the characteristics (e.g., composition and/or volume) of the sample solution remains substantially unchanged as it travels from the inlet to the trap (i.e., the sample solution corresponds to the sample solution received at the inlet). In some embodiments, the microfluidic device is devoid of a processing arrangement between the inlet and the capillary pump (which is typically downstream of the trap).

In some embodiments in which the microfluidic device is devoid of a processing arrangement between the inlet and the trap, the fluid channel contains a tapered or narrowed portion for substantially blocking passage of the species, substantially trapping the species.

In some embodiments, the processing arrangement includes a separator. In some examples, the processing arrangement may consist only of a single separator or a plurality of separators. The separator is arranged to separate content(s) of the sample solution such that the separated content(s) cannot move or travel past the respective separator. The separator may include, for example, a mechanical separator, an electrical separator, a magnetic separator, a chemical separator, or a combination thereof.

In some embodiments, the microfluidic device is devoid of a separator between the inlet and the trap. In some embodiments, the microfluidic device is devoid of a separator between the inlet and the capillary pump (which is typically downstream of the trap). In some embodiments in which the microfluidic device is devoid of a separator between the inlet and the trap, and the fluid channel comprises a tapered or narrowed portion for substantially blocking passage of the species, substantially trapping the species.

In some embodiments, the processing arrangement includes one or more reactors for causing or facilitating reaction of the solution with another solution or a substance. In some examples, the processing arrangement may consist only of a single reactor. The one or more reactors may include a chemical reactor.

In some preferred embodiments, the microfluidic device is devoid of a reactor between the inlet and the trap. In some embodiments, the microfluidic device is devoid of a reactor between the inlet and the capillary pump (which is typically downstream of the trap). In some embodiments in which the microfluidic device is devoid of a reactor between the inlet and the trap, the fluid channel comprises a tapered or narrowed portion for substantially blocking passage of the species, substantially trapping the species.

In some embodiments, the processing arrangement includes one or more samplers for sampling the solution. In some examples, the processing arrangement may consist only of a single sampler.

In some preferred embodiments, the microfluidic device is devoid of a sampler between the inlet and the trap. In some embodiments, the microfluidic device is devoid of a sampler between the inlet and the capillary pump (which is typically downstream of the trap). In some embodiments in which the microfluidic device is devoid of a sampler between the inlet and the trap, the fluid channel comprises a tapered or narrowed portion for substantially blocking passage of the species, substantially trapping the species.

In some embodiments, the processing arrangement includes one or more mixers for facilitating mixing of the solution with another solution or a substance. In some examples, the processing arrangement may consist only of a single mixer.

In some preferred embodiments, the microfluidic device is devoid of a mixer between the inlet and the trap. In some embodiments, the microfluidic device is devoid of a mixer between the inlet and the capillary pump (which is typically downstream of the trap). In some embodiments in which the microfluidic device is devoid of a mixer between the inlet and the trap, the fluid channel comprises a tapered or narrowed portion for substantially blocking passage of the species, substantially trapping the species.

In some embodiments, the processing arrangement includes one or more extractors for facilitating extraction of or for extracting one or more species from the solution. In some examples, the processing arrangement may consist only of a single extractor.

In some preferred embodiments, the microfluidic device is devoid of an extractor between the inlet and the trap. In some embodiments, the microfluidic device is devoid of an extractor between the inlet and the capillary pump (which is typically downstream of the trap). In some embodiments in which the microfluidic device is devoid of an extractor between the inlet and the trap, the fluid channel comprises a tapered or narrowed portion for substantially blocking passage of the species, substantially trapping the species.

In some embodiments, the processing arrangement includes at least one of: the one or more separators, the one or more reactors, the one or more samplers, the one or more extractors, the one or more mixers, etc.

In some embodiments, the trap comprises one or more indicators arranged along at least part of the fluid channel for indicating presence of or an amount of the one or more trapped species. In some embodiments, the one or more indicators may be arranged for indicating presence of or amount (relative or absolute) of one or more target species correlated with the presence of or amount of the one or more trapped species.

In some embodiments, the one or more indicators comprises one or more reference markings, e.g., reference scale markings. In some embodiments, the one or more indicators are for indicating a relative or absolute amount of the one or more trapped species.

In some embodiments, the microfluidic device is portable or handheld. In some embodiments, the microfluidic device is in the form of a chip such as a microchip.

In some embodiments, the microfluidic device includes a device body defining, at least, the inlet and the fluid channel of the trap. In some embodiments, the microfluidic device further includes the capillary pump downstream of the inlet. In an embodiment herein the capillary pump is located downstream of the trap. In some embodiments, the microfluidic device further includes a connector for connecting with the capillary pump, or a space for receiving the capillary pump. In some examples in which the capillary pump includes a microstructure-based capillary pump, the microfluidic device body further defines the microstructure-based capillary pump. In some examples in which the capillary pump comprises a porous-material-based capillary pump, the microfluidic device body further defines a space for receiving, e.g., removably receiving, the porous-material-based capillary pump. In some embodiments, the microfluidic device body is integrally formed. In some embodiments, the microfluidic device body is additively manufactured. In some embodiments, the microfluidic device body is injection molded.

An embodiment of the present invention relates to a method for visually detecting or determining a species in a sample solution by the steps of providing the microfluidic device as described herein, providing a sample solution, the sample solution comprising a species therein, adding the sample solution to the inlet of the microfluidic device, collecting the species in the trap, and visually detecting the species in the trap.

Other features and aspects of the invention will become apparent by consideration of the detailed description and accompanying drawings. Any feature(s) described herein in relation to one aspect or embodiment may be combined with any other feature(s) described herein in relation to any other aspect or embodiment as appropriate and applicable.

Terms of degree such that "generally", "about", "substantially", or the like, are used, depending on context, to account for manufacture tolerance, degradation, trend, tendency, imperfect practical condition, etc. In one example, when a value is modified by terms of degree such as "about", such expression may include the stated value ±15%, ±10%, ±5%, ±2%, or ±1%. The expressions "substantially block" and "substantially trap" mean that the blocking and the trapping are not strictly 100% (small leakage possible due to imperfect practical condition(s), manufacturing tolerance, etc.).

Unless otherwise specified, the terms "connected", "coupled", "mounted" or the like, are intended to encompass both direct and indirect connection, coupling, mounting, etc.

### BRIEF DESCRIPTION OF THE DRAWINGS

Embodiments of the invention will now be described, by way of example, with reference to the accompanying drawings in which:
Figure 1 is a schematic diagram of a microfluidic device for detection of an amount of target species in a sample solution as disclosed in US15/969,877;
Figure 2 is a schematic diagram of the molecules in the sample solution arranged to be placed in the microfluidic device of Figure 1;
Figure 3A is an example of the molecules in Figure 2 in which the target species form the linker for direct measurement;
Figure 3B is an example of the molecules in Figure 2 in which the target species is arranged to react with a target-reactive linker for indirect measurement;
Figure 4 is an example of the molecules in Figure 2 in which the linker is a target oligonucleotide MB155;
Figure 5 is a picture showing a microfluidic device with a visual bar (upper) for a sample solution with the molecules of Figure 4 and a visual bar (lower) for a sample solution without the molecules of Figure 4 as disclosed in US15/969,877;
Figure 6A is a graph showing the length (mm) of accumulated polystyrene micro-particles (PMPs) in the trap of the microfluidic device for different amount (fmol) of target oligonucleotide MB155 (mean ± SEM, n ≥ 5);
Figure 6B is a graph showing the length (mm) of accumulated polystyrene micro-particles (PMPs) in the trap of the microfluidic device for different amount of target oligonucleotide MB155, and which illustrates a linear relationship for 0-60 fmol target oligonucleotide MB155 (mean ± SEM, n ≥ 5);
Figure 7 is a graph showing the length (mm) of accumulated polystyrene micro-particles (PMPs) in the trap of the microfluidic device for different variations of target oligonucleotide MB155, including no MB 155, with MB155, and single-base mutated sequences SNP-A (G at the seventh base from the 5' end replaced with A), SNA-C (G at the seventh base from the 5' end replaced with C), and SNP-T (G at the seventh base from the 5' end replaced with T) (mean ± SEM, n ≥ 6);
Figure 8 is a graph showing the length (mm) of accumulated polystyrene micro-particles (PMPs) in the trap of the microfluidic device for different amount (fmol) of target oligonucleotide MB 155 at 10% v/v serum environment (mean ± SEM, n ≥ 10);
Figure 9 is an example of the molecules in Figure 2 in which the linker is a GR-5 DNAzyme arranged to be cleaved by lead ions (Pb²⁺);
Figure 10 is a graph showing the length (mm) of accumulated polystyrene micro-particles (PMPs) in the trap of the microfluidic device for different concentration (nM) of lead ions (mean ± SEM, n ≥ 5);
Figure 11 is a block diagram of a device for facilitating visual determination of presence and/or quantification of one or more species in a sample solution in some embodiments of the invention;
Figure 12 is a schematic diagram of a microfluidic device in one embodiment of the invention;
Figure 13 is a schematic diagram of a microfluidic device in one embodiment of the invention;
Figure 14 is a schematic diagram of a method for assembling a microfluidic device in Figure 13;
Figure 15 is a microscope image showing an interface between the fluid channel and the filter paper in the microfluidic device of Figure 14;
Figure 16A are microscopic images showing the respective visual bar formed by trapped microparticles trapped by the microfluidic device in Figure 14 (when a solution containing different concentrations of these particles are input into the microfluidic device);
Figure 16B is a graphical representation of the results obtained in Figure 16A;
Figure 17A is a schematic diagram of a device for facilitating visual determination of presence and/or quantification of one or more species in a sample solution in some embodiments of the invention
Figure 17B is a schematic diagram of a device for facilitating visual determination of presence and/or quantification of one or more species in a sample solution in some embodiments of the invention;
Figure 17C is a schematic diagram of a device for facilitating visual determination of presence and/or quantification of one or more species in a sample solution in some embodiments of the invention;
Figure 18A is schematic design for a COVID-19 mucosal antibody test;
Figure 18B is a schematic diagram of a microfluidic device for detection of an amount of unbound PMPs from Figure 18A;
Figure 18C is a standard curve of different antibody concentrations v. PMP accumulations/trapping length in sensitive mode; and
Figure 18D is a standard curve of different antibody concentrations v. PMP accumulations/trapping length in rapid mode.

### DETAILED DESCRIPTION

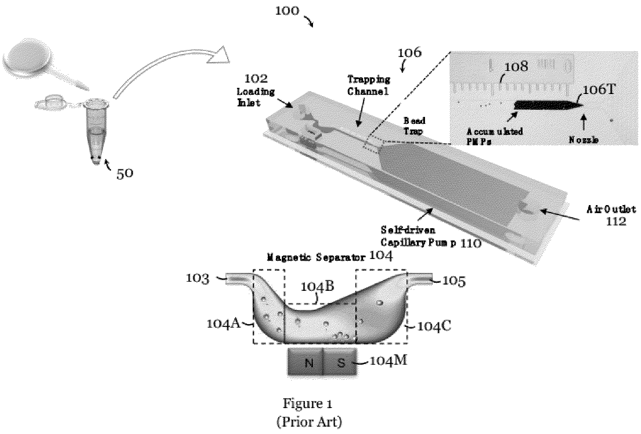

Referring to Figure 1, there is shown a microfluidic device 100, in the form of a microchip, for detection of an amount of target species in a solution 50. The microfluidic device 100 includes a micro-fluid channel with an inlet 102 for loading the sample solution 50. The micro-fluid channel at the inlet 102 tapers to narrow. Downstream of the inlet 102 is a magnetic separator 104 formed by a human stomach-shaped, curved channel portion with a magnet 104M arranged on a side for separating one or more species from the sample solution 50. The magnet 104M is a permanent magnet. More specifically, the channel portion of the magnetic separator 104 is formed by an inlet portion 104A in fluid communication with an upstream fluid channel 103 downstream of the inlet 102, an outlet portion 104C in fluid communication with a downstream fluid channel 105 upstream of the trap 106, and a separation portion 104B arranged between the inlet portion 104A and the outlet portion 104C. The magnet 104M is arranged in a slot on a side of the separation portion 104B for attracting and thereby separating one or more species from the sample solution 50, to provide a sample solution (i.e., a processed solution obtained by processing the sample solution 50 received at the inlet with the magnetic separator 104). The inlet portion 104A extends at an angle to an extension axis of the upstream fluid channel 103 such that the separation portion 104B is offset from the extension axis of the upstream fluid channel 103. The outlet portion 104C extends at an angle to an extension axis of the downstream fluid channel 105 such that the separation portion 104B is offset from the extension axis of the downstream fluid channel 105. The extension axis of the upstream fluid channel 103 and the extension axis of the downstream fluid channel 105 are substantially coaxial. The cross section of the inlet portion 104A at least partly increases from upstream to downstream, and the cross section of the channel at least partly increases from the separation portion 104B to the outlet portion 104C. Further downstream of the separator 104 is the trap 106, formed by another channel portion, for trapping one or more remaining species in the sample solution that has undergone separation, i.e., the sample solution 50 received at the inlet 102 and with one or more species already removed by the magnetic separator 104. The channel portion of the trap 106 includes a tapered portion 106T, with a nozzle, for trapping one or more remaining species. The trap 106 is arranged such that the trapped species is visible for determination of the amount of target species in the sample solution. In particular, the trapped species accumulate in the trap 106 to form a visible bar, like a fuel-gauge display. The length of the bar formed by the trapped species is correlated with the amount of target species in the sample solution. In this example, the channel portion of the trap 106 is transparent or translucent to allow the trapped species to be visible. Reference scale markings 108 are arranged along part of the channel portion of the trap 106 for indicating the amount of the trapped species or the amount of target species in the sample solution. The reference scale markings 108 are provided by a ruler-type instrument. A moving capillary pump, in the form of a self-driven, or non-powered, capillary pump 110, is arranged downstream of the trap 106 to move the sample solution 50 and the sample solution (the processed solution) towards the trap 106. A fluid outlet 112 through which air (and optionally liquid) can escape is arranged downstream of the pump 110.

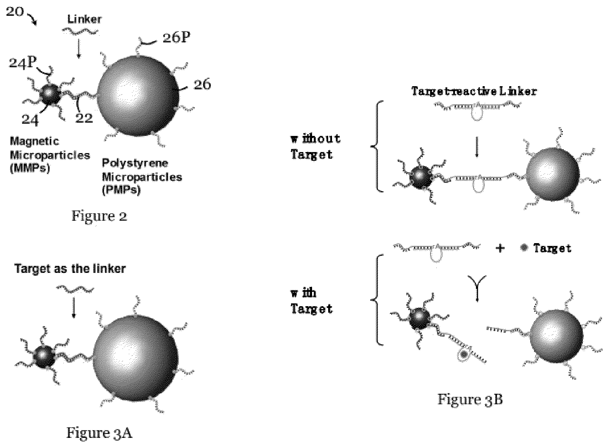

Figure 2 shows the molecules 20 in an example solution arranged to be placed in the microfluidic device 100 of Figure 1. The molecules 20 include a linker 22, as well as magnetic micro-particle (MMP) 24 and a polystyrene micro-particle (PMP) 26, both surface-functionalized with one or more linker-binding probes 24P, 26P, and which can bind with molecular linkers 22 to form a sandwiched structure MMP-linker-PMP.

Figures 3A show an example of the molecules in Figure 2 in which the target species, the amount of which is to be determined, forms the linker for direct measurement. Figure 3B shows an example of the molecules in Figure 2 in which the target species, the amount of which is to be determined, is not part of the sandwiched structure, and the linker is a target-reactive linker suitably arranged to react with the target for indirect measurement. As shown in Figure 3B, the target molecule is arranged to react with the target-reactive linker to cleave the target-reactive linker. Alternatively, the target molecule may form or build a linker.

In operation of the microfluidic device 100 of Figure 1 using solution 50 including the molecules 20 of Figure 2, the sample solution, once loaded into the microfluidic device 100 through the inlet 102, is driven by the self-driven capillary flow to move towards the trap 106. In this example, the fluid channel in the microfluidic device 100 is hydrophilic so that water-based solution could spontaneously flow towards the other end, as moved by capillary force, without the need of external moving or power source. The MMPs-linkers-PMPs then pass into the magnetic separator 104, in which the MMPs-linkers-PMPs (direct measurement with target species being the linker, indirect measurement with target species building the linker) or the MMPs (indirect measurement with target species cleaving the linker) are trapped by electromagnetic interaction with the magnet 104M. By using a stomach-shape micro-channel (Figure 1), the flow first slows down due to the expanding micro-channel at the inlet portion 104A, and the fluid stream is directly guided toward the region with strongest magnetic force in the separation portion 104B. This maximizes the capturing the MMPs-linkers-PMPs or free MMPs. After passing the region of strong magnetic field of the separation portion 104B, the micro-channel further expands to the outlet portion 104C, resulting in a slowed flow before reaching the trap 106. Efficient capturing of MMPs-targets-PMPs or free MMPs can be achieved. Free PMPs in the sample solution can flow downstream and reach the trap 106. The free PMPs accumulate at the trap 106 with a tapered part 106T forming a nozzle sized to prevent downstream flow of PMPs (the width of the nozzle is less than the diameter of the PMPs). Importantly, the depth of the trapping channel portion of the trap 106 is arranged to be slightly larger than the diameter of PMPs such that the PMPs can be accumulated as a monolayer to maximize the visible length of PMP accumulation. The accumulation of PMPs forms a visual bar with a quantifiable length, readily observable by the naked eye, optionally with the assist of reference markings 108. The length of the bar is correlated with the amount of target molecules. For example, in direct measurement, the target molecules can be the linker such that the PMP accumulation is inversely proportional to the amount of target molecules. In indirect measurement, the target molecules can interact with the linker, e.g. forming or degrading the linker, such that the PMP accumulation is proportional to the amount of target molecules. During trapping, the water-based buffer solution can pass through the nozzle. Importantly, the capillary pump 110 is placed to ensure capillary attraction of liquid wicking until the capillary pump 110 is fully filled. This may ensure a consistent volume filling the micro-channel, minimizing fluctuations and errors in measurement.

It should be appreciated that the connection of the molecules in Figure 2 can be formed in different ways depending on the type of the linkers. For example, for direct measurement of single-strand DNA oligonucleotides, the linker-binding probes can be designed as a pair of single-strand oligonucleotides with sequences complementary to that of the target in juxtaposition. Alternatively, for direct measurement of protein, the linker-binding probes may be a pair of antibodies that recognize two different antigenic epitopes of the protein. On the other hand, for the indirect measurement where target molecules interact with the linker, the linker can be DNAzyme, a form of DNA oligonucleotide that could be cleaved with the presence of target molecules such as metal ions. In one example, to immobilize the linker-binding probes, streptavidin-coated MMPs and PMPs are used to immobilize the biotinylated probes. After immobilization, the MMPs and PMPs were rinsed three times to remove unused probes. For each washing step, the MMPs are collected using a magnetic separation rack, while the PMPs are collected using a centrifuge. For direct measurement, the target molecules can be directly mixed with MMPs and PMPs with gentle shaking. For indirect measurement, the target can be first mixed and react with the linker, followed by mixing with MMPs and PMPs.

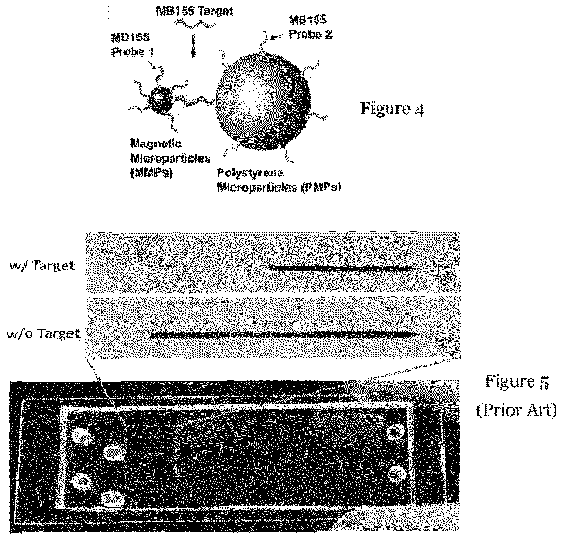

Figure 4 shows a specific target oligonucleotide, i.e. MB155, the amount of which is to be measured using the microfluidic device of Figure 1. As shown in Figure 4, two probes of biotinylated oligonucleotides (MB155 probe 1 and probe 2) were designed with a sequence complementary to MB155 in juxtaposition. MB155 probe 1 and probe 2 were respectively immobilized onto streptavidin-coated MMPs and PMPs via a biotin-streptavidin complex. As such, when the MB155 target is present, probe 1 and probe 2 will simultaneously hybridize with the MB155 target in juxtaposition, forming a sandwich-type aggregate, MMPs-targets-PMPs. In this example, to prevent the inertial forces from breaking the linkage between MMPs and PMPs, MMPs with a diameter of 0.36 µm were selected, such that multiple MMPs can attach onto one PMP to disperse the force. The number of free PMPs trapped can be used to represent the amount of MB155 targets. After rinsing, probe 1-modified MMPs and probe 2-modified PMPs were mixed with MB155, and the mixture was loaded to the inlet. As the microchannel is hydrophilic after plasma treatment, this water-based solution would spontaneously flow towards the other end, driven by capillary force without the need for an external power source. In this example, the narrowing nozzle of the trap 106 has a minimum width of 8 µm, which can block the PMPs with a diameter of 15 µm. The depth of the PMP trapping channel was designed to be 25 µm, such that the PMPs can be accumulated as a monolayer to maximize the visible length of PMP accumulation.

Figure 5 shows visual bars for a sample solution with the MB155 target (upper) and a blank sample (lower). As shown in Figure 5, a visible bar of accumulated PMPs can be observed in the channel portion of the trap 106, and its length can be quantified using a dipstick-type ruler with reference measurement markings 108. For the blank sample, i.e., without target linkers, only MMPs were captured in the magnetic separator and all PMPs suspended in solution flow towards the channel of the trap 106, giving rise to a longer PMP accumulation length. For the sample with the MB155 target, a portion of PMPs would be retained at the site of the magnetic separator 104, and so the length of PMP accumulation is shortened.

During trapping, the water-based buffer solution can penetrate through the gap between the PMPs and the nozzle at the trap 106. Importantly, the capillary pump 110 was placed after that to ensure the capillary attraction of liquid wicking until the capillary pump 110 is fully filled (volume capacity: 4.07 µl), which ensures a consistent volume fill in the micro-channel and minimizes the data fluctuation between each experiment.

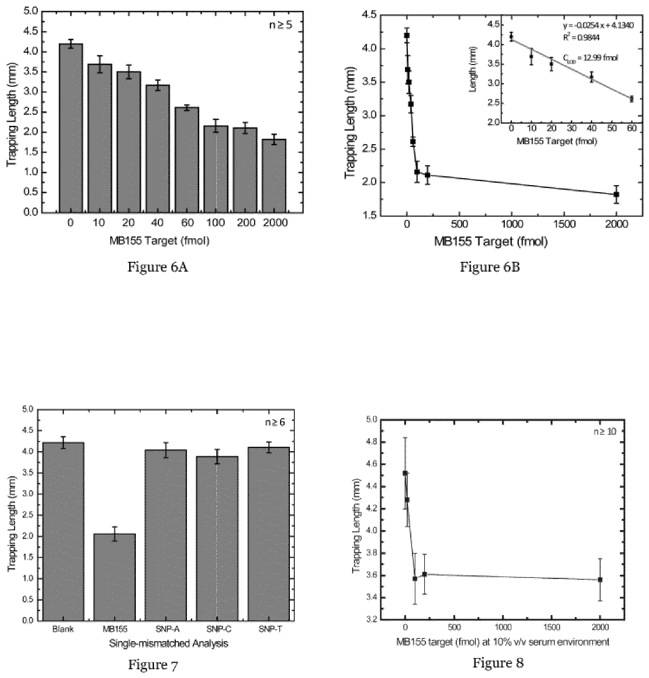

The limit of detection of oligonucleotides in further determined using different amount of the MB155 target, i.e. 0 mol, 10 fmol, 20 fmol, 40 fmol, 60 fmol, 100 fmol, 200 fmol and 2000 fmol in 20 µl. Figure 6A illustrates the length of accumulated PMPs in the channel of the trap 106 for different amount of MB155 targets (which reflects the amount of free PMPs escaping from the magnetic separator). As shown, the PMP accumulation was inversely proportional to the amount of the target molecules. For the blank sample (0 mol), most of the PMPs can escape from the magnetic separator and accumulate in the channel of the trap 106, resulting in the longer visible bar. In contrast, when the amount of target molecules increases, the length of the visible bar decreases. Figure 6B also shows the length of PMP accumulation with respect to the amount of the target species. As shown in Figure 6B, the linear range of such change is from 0 mol to 60 fmol (inset in Figure 6B), and the limit of detection is determined to be 13 fmol (0.65 nM in 20 µl, S/N = 3, calculated on the basis of 3σ/k, where σ is the standard deviation of the blank sample, and k is the absolute slope of the linear equation, -0.0254). This demonstrates a performance comparable to that of UV/vis spectrometry.

The selectivity of detection in the microfluidic device 100 is also investigated using singlenucleotide polymorphisms (SNP). The selectivity is crucial for differentiating non-specific hybridization. In the test, the seventh base of the MB 155 target from the 5' end, G, was replaced by A, T and C, and denoted as SNP-A, SNP-T, and SNP-C, respectively. Using 200 fmol (10 nM in 20 µl) for each case, the length of PMP accumulation shows that the results of SNP-A, SNP-T and SNP-C almost had no difference with that of the blank sample (0 mol) (around 4.20 mm in length), as shown in Figure 7. For the MB 155 target, the length was significantly shorter (2.00 mm). This shows that the microfluidic-based trap can be used for differentiating singlenucleotide polymorphisms.

Next, the compatibility of the microfluidic device 100 is evaluated in a complex bio-fluid. Blood serum contains proteins, cells, RNAs, and DNase/RNase, and such interfering materials may lead to considerable challenges for hybridization and even cause failure of device functionality. To investigate the tolerance to such a complex environment, a 10% v/v serum environment containing varied amount of MB155 (0 mol, 10 fmol, 20 fmol, 40 fmol, 60 fmol, 100 fmol, 200 fmol and 2000 fmol in 20 µl) without any further pre-treatment was used. Similar to the results obtained in the buffer solution, as shown in Figure 8, the length of PMP accumulation decreased with an increase of the target amount. While the overall length increased slightly because a small number of MMPs-targets-PMPs were carried to the trap 106 due to the high viscosity of serum, the limit of detection is around 20 fmol. This demonstrates the compatibility with a complex bio-fluid and the ability to tolerate the interfering materials in such a microfluidic device 100.

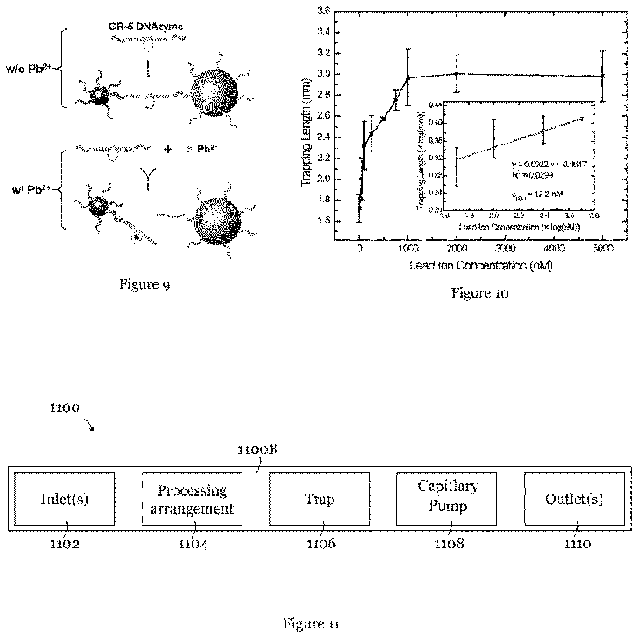

Based on the characterization above, a model application that uses detection of oligonucleotides for monitoring lead ions was tested. On the basis of nucleic acid hybridization, it was reported that DNAzyme, a form of DNA oligonucleotide, could catalyze a specific hydrolytic cleavage in the presence of lead ions Pb²⁺. Accordingly, the target oligonucleotide MB155 is replaced with GR-5 DNAzyme, which exhibits a high selectivity towards Pb²⁺. As shown in Figure 9, the DNAzyme consists of a substrate strand GRDS and an enzyme strand GRE and the two termini of GRDS were extended and designed to be able to hybridize with MB155 probe 1 and probe 2, respectively. As such, if Pb²⁺ is absent, the GR-5 DNAzyme at the amount of 200 fmol (10 nM in 20 µl) can connect MMPs and PMPs, leading to the formation of a sandwiched structure, MMPs-DNAzyme-PMPs. In contrast, when Pb²⁺ is present, the ions cleave the substrate strand at the RNA site (rA) into two parts through a phosphodiester bond cleavage. This prevents the formation of the sandwiched structure MMPs-DNAzyme-PMPs and allows more PMPs to accumulate in the channel of the trap 106.

Different concentrations of Pb²⁺ solution, including 0 nM, 50 nM, 100 nM, 250 nM, 500 nM, 750 nM, 1000 nM, 2000 nM and 5000 nM, was tested. As expected, the trapping length due to PMP accumulation reflected the concentrations of Pb²⁺ with a positive proportion, as shown in Figure 10. Moreover, using the log scale, the linear range of such a change was from 0 to 250 nM (inset in Figure 10), and the limit of detection can be determined to be 12.2 nM (S/N = 3, calculated on the basis of 3σ/k, where σ is the standard deviation of the blank sample, and k is the absolute slope of the linear equation, 0.0922). Remarkably, the limit of detection is below the maximum human exposure to drinking water, 48 nM (10 µg 1-1), according to the guideline of the World Health Organization. As such, with sufficient sensitivity and the ease of intensive sample preparation when applied in drinking/clean water, the results demonstrate a feasible application for monitoring lead ion contamination.

The above disclosure provides a simple and low-cost microfluidic platform that forms a quantitative visual bar visible by naked eyes. In the illustrated example, the detection principle is based on the changed connectivity between MMPs and PMPs. Thus, by selecting appropriate linker-binding probes, and/or combining the use of linker that reacts with the target molecules, the above disclosure can be applied to various target molecules, such as nucleic acids, protein, metal ion, or chemical compounds. As such, the application of the above disclosure is diverse, including inorganic chemistry, diseases diagnosis, and environmental toxin screening.

In one application, the above disclosure can be used for the detection of disease-related biomarkers, such as nucleic acid or protein based biomarkers. Recently, nucleic acid based biomarkers have shown importance for cancer diagnosis. MicroRNAs, small (~22 nt) regulatory RNAs present in blood stream due to dysfunction of cancer, was recently found its promise as tissue-based markers for cancer classification and prognostication. Thus, microRNAs can be effectively detected by using the above example to connect MMPs and PMPs, which offers unprecedented approach to identify and evaluate the risk of cancer at early stage. Similarly, for protein-based biomarkers, by using a pair of antibodies on MMPs and PMPs that recognize two different antigenic epitopes, the presence of protein biomarkers can result in a connection between MMPs and PMPs. Malaria, for example, can be detected via Plasmodium falciparum histidine rich protein (Pf HRP II), the biomarker produced by malariacausing parasite in patients' serum.

In another application, the above disclosure can be used for environmental monitoring. Botulinum neurotoxin (BoNT) is considered one of the world's most dangerous toxins. Intoxication by BoNT may occur through the ingestion of contaminated food or from biological weapons in some undeveloped countries or military areas. Due to its toxicity, remarkable stability, and persistence in the body, a very low dose of BoNT can cause muscle paralysis or even death. To detect this species, SNAPtide can be used to connect MMPs and PMPs. SNAPtide is a short peptide designed to mimic the synaptosomal-associated protein25 (SNAP-25, a component of the trans-SNARE complex) with a site cleavable by BoNT. Thus, as an indirect measurement, the presence of BoNT would cleave the SNAPtide, causing an increase of free PMPs and longer PMP accumulation length.

In yet another application, the above disclosure can be used for monitoring water safety. Lead contamination in drinking water has been a serious problem worldwide because of the wide use of lead pipes in plumbing. Such contamination causes significant concerns of water safety in public health. Particularly, children with lead poisoning may suffer from slow development of childhood behaviors and permanent intellectual disability, creating significant threats to their well-being. Thus, it is highly desired to provide simple and portable platforms to every enduser for continuous monitoring of lead contamination in their domestic water source. By using DNAzyme that reacts with the lead ion to connect MMPs and PMPs, the presence of lead ion can cleave the DNAzyme, causing an increase of PMP accumulation in the trap.

Examples of the above disclosure enable ready visual detection and measurement of various types of chemical and biochemical molecules, e.g., nucleic acids, protein, chemical compounds, metal ions, and thus have great potential in different applications such as portable biosensors, medical diagnosis, environmental safety, and scientific research. The above disclosure provides a readily accessible platform ranging from disease diagnosis to monitoring of environmental toxin and metal contamination. The above disclosure is well applicable to healthcare and environmental monitoring, especially in resource-limited settings.

Examples of the above disclosure also provide various advantages. The trap, e.g., a narrowing nozzle, can trap and accumulate PMPs that form a quantitative visual bar readable by naked eyes. This provides improved convenience when compared with using a bulky reader for fluorescence or color absorbance. The accumulation of PMPs can be directly visualized and readily quantified by its length, optionally with calibration, to provide accurate result and effective measurement.

Also, the procedure of the assay in the above disclosure is simple. The use of capillary flow promotes convenient and economical operation. The MMPs and PMPs can be modified with linker-binding probes in advance, to tailor for different applications. After mixing the sample with linker and surface-functionalized MMPs and PMPs, the only experimental procedure is to dispense the mixture into the microchip. As such, the capillary flow and magnetic attraction by permanent magnet can achieve an automated operation in a substantially power-free manner and without the need of additional instruments. There is also no need for a special environment or experimental control. As a result, the requirement of experimental procedure is minimized.

Furthermore, the type of target species (the amount of which is to be measured) can be diverse. As aforementioned, the measurement can be direct, i.e. the target molecules can be the linker to connect MMPs and PMPs, or indirect, i.e. the target molecules can interact with the linker to alter connection between MMPs and PMPs. Thus, diverse types of targets can be used as long as the force change is sufficient to induce different connection between MMPs and PMPs. With particular advantages for portability, energy efficiency, and user-friendly interface, examples of the above disclosure can facilitate portable testing for point-of-care devices.

Examples devices of the above disclosure are provided to provide background understanding. Certain embodiments of the present invention are now presented with reference to Figures 11 to 16B. Some embodiments of the present invention can be considered as alternative to or improvement of the example's devices of the above disclosure.

Referring to Figure 11, there is shown a device 1100, preferably a microfluidic device, which may be in the form of a microchip, for facilitating visual determination of presence and/or quantification of a species in a sample solution. The species may be in the form of particles, e.g., microparticles, nanoparticles, etc.

The microfluidic device 1100 includes an inlet 1102 for loading or inputting a solution to the microfluidic device 1100. The inlet 1102 may be provided by a fluid channel, such as a micro-fluid channel. As an example, the inlet 1102 may be the same or similar to the inlet 102 of Figure 1. In some embodiments, the microfluidic device 1100 can have multiple inlets for loading, inputting or otherwise receiving solution(s).

In some embodiments, the microfluidic device 1100 also includes a processing arrangement 1104. The processing arrangement 1104 may be arranged either upstream or downstream of the inlet 1102, or the processing arrangement 1104 may include the inlet 1102. The processing arrangement 1104 is arranged to process the sample solution received at the inlet 1102, e.g., to alter one or more characteristics (e.g., composition, volume, etc., or any combination thereof) of the sample solution received at the inlet 1102, to form a processed solution. The processing arrangement 1104 may include physical means, chemical means, etc., or any combination thereof, arranged to alter the one or more characteristics of the sample solution received at the inlet 1102. In some examples, the processing arrangement 1104 may include one or more separators. The separator(s) may include a mechanical separator, a chemical separator, an electric separator, a magnetic separator (e.g., the magnetic separator 104 of Figure 1), etc. The separator(s) may be used for separating one or more species from the sample solution. In some examples, the one or more separators may stop the separated specie(s) from moving past the processing arrangement 1104. In some examples, the processing arrangement 1104 may include, additionally or alternatively, one or more reactors (e.g., one or more chemical reactors) for causing or facilitating reaction of the sample solution with another solution or a substance. In one example, the reactor(s) include further inlet(s) for receiving solution(s) for reacting with the sample solution received at the inlet 1102, and the further inlet(s) may be in fluid communication with the inlet 1102 via fluid channel(s). In some examples, the processing arrangement 1104 may include, additionally or alternatively, one or more samplers for sampling of the sample solution. In some examples, the processing arrangement 1104 may include, additionally or alternatively, one or more extractors for facilitating extraction of or for extracting one or more species from the sample solution received at the inlet 1102. In some examples, the one or more extractors may stop the extracted specie(s) from moving past the processing arrangement 1104. In some other examples, the one or more extractors may enable the extracted specie(s) to move past (downstream of) the processing arrangement 1104. In some examples, the processing arrangement 1104 may include, additionally or alternatively, one or more mixers for facilitating mixing of the sample solution received at the inlet 1102 with another solution or a substance. In embodiments in which the processing arrangement 1104 includes at least reactor(s) and separator(s), the reactor(s) may be arranged upstream of, downstream of, or parallel to the separator(s). In embodiments in which the processing arrangement 1104 includes at least sampler(s) and separator(s), the sampler(s) may be arranged upstream of, downstream of, or parallel to the separator(s). In embodiments in which the processing arrangement 1104 includes at least reactor(s) and sampler(s), the reactor(s) may be arranged upstream of, downstream of, or parallel to the sampler(s). Effectively, the processing arrangement 1104 is arranged to process the sample solution received at the inlet 1102 to provide a processed solution.

The microfluidic device 1100 also includes a trap 1106 downstream of the processing arrangement 1104, or if no processing arrangement is present, downstream of the inlet 1102. The trap 1106 may be in fluid communication with the inlet 1102, via any suitable fluid communication arrangement (e.g., fluid channel(s)). The trap 1106 includes, at least, a fluid channel, such as a micro-fluid channel, for facilitating substantially trapping the species of the sample solution (the sample solution may be the sample solution received at the inlet 1102, if no processing arrangement 1104 is present; or the sample solution may be the processed solution processed by the processing arrangement 1104, if processing arrangement 1104 is present). In some examples, the species of the sample solution may be a species present in the sample solution received at the inlet 1102. In some examples, the species of the sample solution may be a species obtained at a result of processing of the sample solution by the processing arrangement 1104 (i.e., the species may not be initially present in the sample solution received at the inlet 1102). The species are preferably in the form of particles (e.g., microparticles). The trap 1106 is arranged such that the one or more trapped species is visible (e.g., directly visible or their shadow being visible) for visual determination of presence and/or quantification. The fluid channel of the trap 1106 may be straight, or curved, depending on embodiments. The fluid channel is preferably transparent (or translucent) so that the one or more trapped species (or their shadow) is visible. In some embodiments, the fluid channel of the trap 1106 includes a narrowed or tapered portion, forming a nozzle, for substantially blocking passage of the species, substantially trapping the species. The nozzle may be sized such that its narrowest width is less than diameters or an average diameter of the particles in the sample solution. In some embodiments, the fluid channel may lack narrowed or tapered portion for substantially blocking passage of the species, substantially trapping the species. In these embodiments, the trap includes other means to substantially block passage hence substantially trapping the species. In some embodiments, one or more indicators may be arranged along at least part of the fluid channel for indicating presence of or an amount of the one or more trapped species. The indicator(s) may be in the form of number(s), alphabet(s), symbol(s), character(s), shape(s), word(s), etc. The indicator(s) may be reference marking(s), such as reference scale marking(s), which may be provided by a ruler-type or ruler-like arrangement. The indicator(s) or reference marking(s) may be used to indicate relative or absolute amount of the one or more trapped species. In one example, the one or more trapped species, depending on their amount presence in the sample solution, may accumulate in the trap 1106 to form a visible bar, like a fuel-gauge display. The length of the bar formed by the one or more trapped species is correlated with an amount (relative or absolute) of one or more target species (different from the one or more trapped spices in the sample solution). The indicator(s) may facilitate ready read-out of the relative or absolute amount of the one or more target species and/or the relative or absolute amount of the one or more trapped species.

The microfluidic device 1100 also includes a capillary pump 1108 for facilitating or causing movement of the sample solution (which may be the sample solution received at the inlet 1102, if no processing arrangement 1104 is present; or the processed solution processed by the processing arrangement 1104, if processing arrangement 1104 is present) towards the trap 1106.

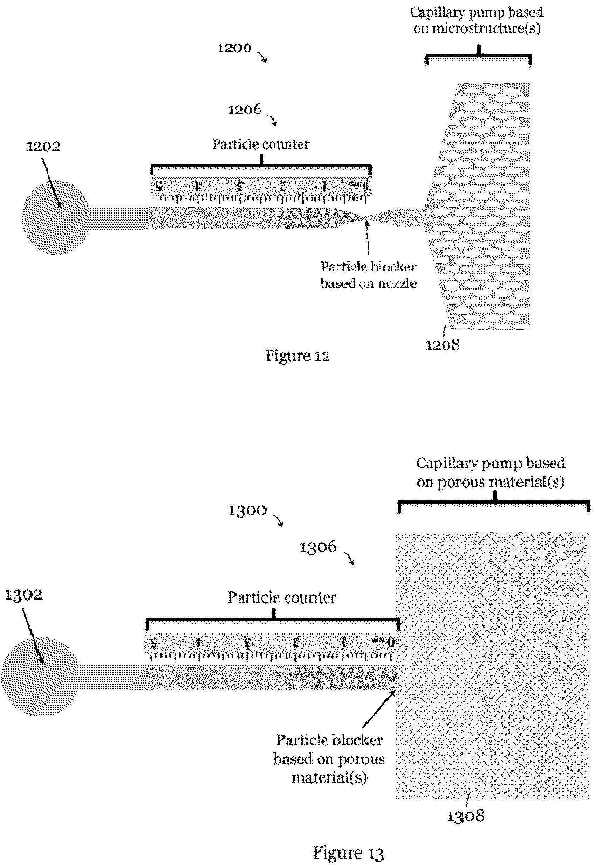

The capillary pump 1108 is in fluid communication with the fluid channel of the trap 1106. The capillary pump 1108 may include a microstructure-based capillary pump or a porous-material-based capillary pump. A microstructure-based capillary pump may include a microchannel with a series of microstructures into which the sample solution is drawn via surface tension/adhesion-type capillary action. A porous-material-based capillary pump may include a filter, e.g., filter paper, into which the liquid is drawn via surface tension/adhesion-type capillary action and may be further drawn by absorption-type capillary action. The capillary pump is preferably self-driven (by capillary action) without the need of a power source. In some embodiments, the capillary pump is operable to substantially block passage of and substantially trapping the species, preferably in at least part of the fluid channel and/or the trap (see, Figure 13). In some of these embodiments, the capillary pump is a microstructure-based with gap size or an average gap size smaller than a diameter or average diameter of the one or more species (see, Figure 12). In some of these embodiments, the capillary pump includes porous-material-based capillary pump and is made of porous material(s) with pore size(s) or an average pore size smaller than a diameter or average diameter of the one or more species (see, Figure 13).

The microfluidic device 1100 also includes an outlet 1110 through which fluid (e.g., gas, liquid, etc.) can escape. The outlet 1110 is in fluid communication with the capillary pump 1108 and is arranged downstream of the capillary pump 1108. In some embodiments, the microfluidic device 1100 can have multiple such outlets.

The microfluidic device 1100 may include a device body 1100B. The microfluidic device body 1100B may be additively manufactured or injection moulded. The microfluidic device body 1100B may define, at least, the inlet 1102, the fluid channel of the trap 1106, and the outlet 1110. The microfluidic device body 1100B may further define at least part of the processing arrangement 1104. Additionally or alternatively, the microfluidic device body 1100B may define the capillary pump 1108. In some examples in which the capillary pump 1108 includes a microstructure-based capillary pump, the microfluidic device body further defines the microstructure-based capillary pump (microstructure / microchannel). In some examples in which the capillary pump 1108 includes a porous-material-based capillary pump, the microfluidic device body 1100B may further define a space for receiving, e.g., removably receiving, the porous-material-based capillary pump (e.g., filter).

Figure 12 shows a microfluidic device 1200 in one embodiment of the invention. The microfluidic device 1200 is an example implementation of the microfluidic device 1100. The microfluidic device 1200 includes an inlet 1202 arranged to receive a solution, and a trap 1206 in fluid communication with the inlet 1202 and arranged to substantially trap the species from the sample solution (in this case, the microfluidic device 1200 is devoid of a processing arrangement so the sample solution is the sample solution received at the inlet 1202), and a capillary pump 1208 downstream of the trap 1206 for causing or facilitating movement of the sample solution towards the trap 1206. In this example, the species are in the form of microparticles. The trap 1206 includes a fluid channel and is arranged such that the trapped species is visible for visual determination of presence and/or quantification. In this embodiment, the fluid channel of the trap 1206 includes a converged portion / nozzle with a width less than diameters or average diameter of the microparticles. The converged portion / nozzle can act as a particle blocker. In this embodiment, reference markings are arranged along at least part of the fluid channel for indicating an amount of the one or more trapped species (e.g., an accumulated length of the trapped species in the fluid channel). In this example, the reference markings begin from the converged portion / nozzle, and extend away from the capillary pump 1208. The reference markings can act as a particle counter. In this embodiment, the capillary pump 1208 includes a capillary pump based on microstructure. In this embodiment, the microfluidic device 1200 is devoid of a processing arrangement (separator / reactor / sampler / mixer / extractor) between the inlet 1202 and the fluid channel / the trap 1206.

Figure 13 shows a microfluidic device 1300 in one embodiment of the invention. The microfluidic device 1300 is an example implementation of the microfluidic device 1100. The microfluidic device 1300 includes an inlet 1302 arranged to receive a solution, and a trap 1306 in fluid communication with the inlet 1302 and arranged to substantially trap a species of the sample solution (the sample solution received at the inlet 1302, if no processing arrangement exists; the processed solution processed by the processing arrangement, if processing arrangement exists). In this example, the species are in the form of microparticles. The trap 1306 includes a fluid channel and is arranged such that the trapped species is visible for visual determination of presence and/or quantification. In this embodiment, the fluid channel of the trap 1306 lacks converged portion or nozzle. In this embodiment, a capillary pump 1308 is arranged downstream of and immediately adjacent trap 1306 and the fluid channel. The capillary pump 1308 causes or facilitates movement of the sample solution towards the fluid channel or more generally the trap 1306. In this embodiment, the capillary pump 1308 includes a porous-material-based capillary pump provided by a filter paper. The filter paper is made of porous material(s) with an average pore size smaller than diameters or average diameter of the microparticles. The filter paper or the porous-material-based capillary pump can substantially block passage of and substantially trap the species in the fluid channel. The filter paper or the porous-material-based capillary pump can act as a particle blocker. In this embodiment, reference markings are arranged along at least part of the fluid channel for indicating an amount of the one or more trapped species (e.g., an accumulated length of the trapped species in the fluid channel). In this example, the reference markings begin from the interface of the capillary pump 1308 and the fluid channel, and extend away from the capillary pump 1308. The reference markings can act as a particle counter. Although not illustrated, in this embodiment, the microfluidic device 1300 may or may not include processing arrangement (separator(s) / reactor(s) / sampler(s) / mixer(s) / extractor(s)) between the inlet 1302 and the fluid channel / the trap 1306.

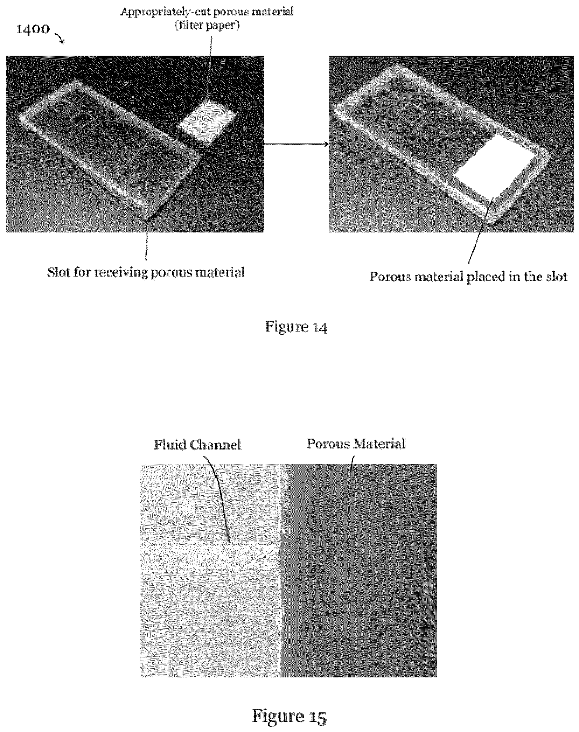

Figure 14 is a schematic diagram of a method for assembling the microfluidic device 1400 (as fabricated in accordance with the design of Figure 13). The microfluidic device 1300 includes a body defining the inlet, the fluid channel of the trap, and an outlet. The microfluidic device also defines a space for receiving the porous material (e.g., filter paper) which acts as the capillary pump. In this embodiment, the body is additively manufactured using plastic material(s). Figure 14 shows a slot on the body for receiving the porous material (e.g., filter paper), and the placement or insertion of the porous material into the slot.

Figure 15 is a microscopic image showing the interface between the capillary pump which in this case is a porous material, and more specifically a piece of filter paper, and the fluid channel in the microfluidic device (see Figure 14, at 1400), which illustrates the alignment between the porous material and the fluid channel (of the trap).

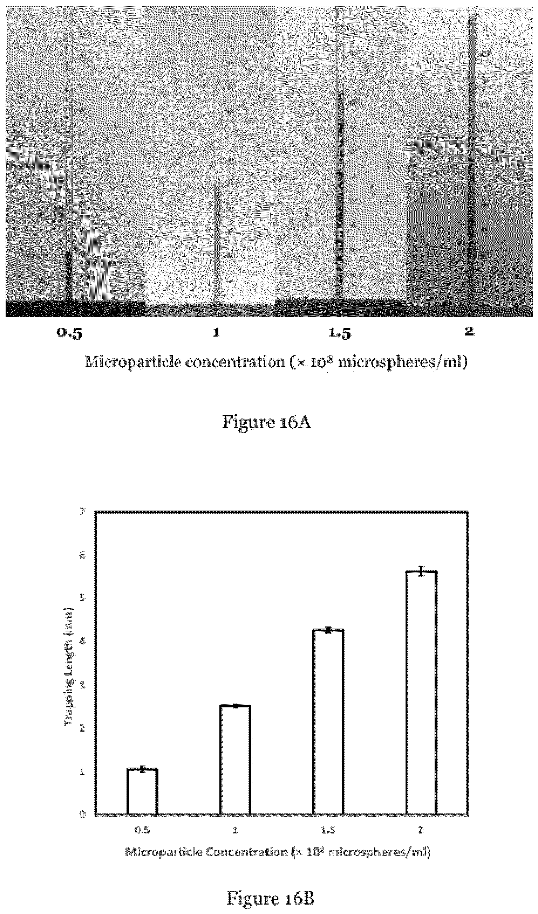

Figure 16A contain microscopic images showing respective visual bar formed by trapped microparticles trapped by the microfluidic device 1400 in Figure 14 when solution containing different concentrations of these particles are input into the microfluidic device. The concentrations used in the tests include 0.5 × 10⁸, 1 × 10⁸, 1.5 × 10⁸, and 2 × 10⁸ microspheres/ml respectively. Figure 16B is a graphical representation of the results obtained in Figure 16A (n = 3). The microparticles used in this experiment are 15.3 µm (average) diameter polystyrene particles purchased from Bangs Laboratory Inc. (USA). The tested concentrations are achieved by diluting the microparticles with the stock buffer which is a 0.1M MES in DEPC treated water. The experiment is carried out by injecting 1 µL of microparticle solution into the microfluidic device. The concentrations used in this experiment is determined by referencing a reference experiment that indicates that 1 µL of 2 × 10⁸ microspheres/ml will give a trapping length of the entire trap region.

In the example of Figures 16A and 16B, the indicators are reference marks spaced generally equally and arranged along the fluid channel.

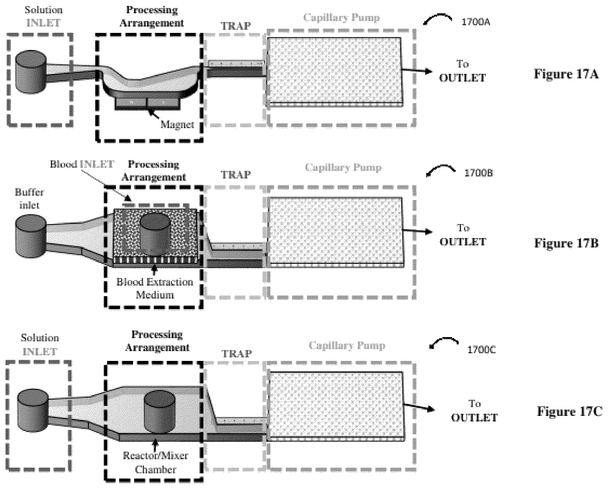

Figures 17A to 17C illustrate further example devices 1700A, 1700B, 1700C in some embodiments of the invention. It should be appreciated that the microfluidic devices 1700A, 1700B, 1700C are only example implementations of the microfluidic device 1100 and that the microfluidic device 1100 may be implemented differently.

Figure 17A shows a device 1700A for facilitating visual determination of presence and/or quantification of a species in a sample solution in some embodiments of the invention. In this embodiment, the microfluidic device 1700A includes an inlet (solution inlet) for receiving a solution. The microfluidic device 1700A also includes a processing arrangement in fluid communication with the inlet. The processing arrangement in this example is in the form of a magnetic separator (e.g., a magnet) for separating one or more species from the sample solution received at the inlet to provide a sample solution (i.e., a processed solution). The microfluidic device 1700A further includes a trap in fluid communication with the inlet and arranged to substantially trap one or more species (particles) of the sample solution. In this example, the trap includes a fluid channel and is arranged such that the one or more trapped species is visible for visual determination of presence and/or quantification. In this example, the fluid channel of the trap lacks a converged portion or nozzle and contains a counter. In this example, the trap further includes a capillary pump (a.k.a. the source), arranged downstream of and immediately adjacent the fluid channel, for causing or facilitating movement of the sample solution towards the fluid channel or more generally the trap. In this example, the capillary pump includes a porous-material-based capillary pump made of porous material(s) with an average pore size smaller than the diameters of, or average diameter of the particles (e.g., the one or more species). The porous-material-based capillary pump can substantially block passage of and substantially trap the species in the fluid channel to form a trapped species. The porous-material-based capillary pump can act as a particle blocker. In this example, reference markings are arranged along at least part of the fluid channel for indicating an amount of the one or more trapped species (e.g., an accumulated length of the trapped species in the fluid channel). The reference markings can act as a particle counter. In this example, an outlet in fluid communication with the trap is arranged further downstream of the trap.

Figure 17B shows a device 1700B for facilitating visual determination of presence and/or quantification of a species in a sample solution in some embodiments of the invention. In this embodiment, the microfluidic device 1700B includes an inlet for receiving a buffer solution and an inlet (blood inlet) for receiving a blood solution. The microfluidic device 1700B also includes a processing arrangement in fluid communication with the inlets. The processing arrangement in this example is in the form of a blood serum extractor for extracting blood serum from the blood solution received at the inlet, to provide a sample solution (i.e., a processed solution). To this end, in this example, the extractor includes a blood extraction medium for facilitating extraction of the blood serum (liquid portion of the blood without cells and clotting factors). The extracted blood serum and the buffer solution are allowed to flow past the processing arrangement, and they correspond to the sample solution. The microfluidic device 1700B further includes a trap in fluid communication with the inlet and arranged to substantially trap one or more species (particles) of the sample solution. The trap contains a counter. These one or more species (particles) may be, for example, microparticles modified with entities (protein and/or DNA) that capture target biomarkers from the blood plasma (i.e., viral proteins, mRNAs, and DNA). The trap and outlet arrangement in this example is similar to the trap and outlet arrangement in the microfluidic device 1700A of Figure 17A so details will not be repeated here. In some other embodiments, the trap may instead be the trap of Figure 12. The capillary pump is arranged directly downstream of the counter portion of the trap.

Figure 17C shows a device 1700C for facilitating visual determination of presence and/or quantification of a species in a sample solution in some embodiments of the invention. **In** this embodiment, the microfluidic device 1700B includes an inlet for receiving a solution and an inlet (reactant inlet) for receiving a reactant (solution). The microfluidic device 1700C also includes a processing arrangement in fluid communication with the inlets. The processing arrangement in this example is in the form of a reactor/mixer chamber for facilitating and/or inducing a reaction/mixing of the sample solution and reactant (e.g., two or more species), to provide a sample solution (i.e., a processed solution). To this end, in this example, the reactor/mixer includes the reactant inlet and a reactor/mixer chamber. The sample solution and reactant, after reaction/mixing, becomes the sample solution. The microfluidic device 1700B further includes a trap, containing a capillary in fluid communication with the inlet and arranged to substantially trap one or more species (particles) of the sample solution. The trap in Figure 17C contains an optional counter. The trap and outlet arrangement in this example is similar to the trap and outlet arrangement in the microfluidic device 1700A of Figure 17A so details will not be repeated here. In some other embodiments, the trap may instead be the trap of Figure 12.

Microparticles are used extensively in various fields such as biomedical engineering, from drug delivery, to biosensors, to diagnostic devices. Some embodiments of the invention have provided a relatively simple microfluidic device which enables the quantification of microparticles based on their geometrical properties without the use of extra or excessive laboratory equipment.

Some embodiments of the invention have provided a relatively simple microfluidic device that can be used to capture and quantify microparticles based on their geometrical properties, e.g., their diameter. Some embodiments of the invention have provided a microfluidic device with three main parts: a particle counter, a trap, and a capillary pump, which works in tandem with each other. In some embodiments of the invention, when the microparticles (in a solution) are introduced into the microfluidic device, the microparticles will be substantially captured and trapped in place by an appropriately designed particle blocker (e.g., one with geometry smaller than the diameters or average diameter of the microparticles). As the liquid flow in the microfluidic device continues, the microparticles will be stacked or accumulate in a microchannel. Indicator(s) along the channel provide a particle counter which enables observable quantification of the accumulative length of the stacked microparticles, for quick read-out or on-site measurement. In some embodiments of the invention, the overall flow of the microfluidic device is driven by a capillary pump which exhibits via wicking/capillary action, a negative pressure to create a pressure difference between the inlet and the outlet of the microfluidic device. This can facilitate a self-driven fluid flow across the microfluidic device, from inlet towards outlet, without the need of an external capillary pump. The particle blocker may be presented in different forms in different embodiments. One example form is to design the nozzle as a converging microchannel with the narrowest width less than the diameters or average diameter of the microparticles. In this example, an appropriately designed capillary pump that may include porous materials and/or a microchannel with a series of microstructures can be used to facilitate the fluid flow. In another example, it utilizes a porous material(s) with an average pore size smaller than the diameter of the microparticles, to substantially block passage of microparticles in a manner similar to the converging nozzle described herein. Advantageously, in this example, the porous material may also serve as a capillary pump to facilitate liquid flow. The resulting design can thus be made simpler, more compact, and/or cost efficient.

Some embodiments of the invention have provided a simple method to measure and quantify microparticles by the means of an appropriate geometrically designed particle counter, a trap, and capillary pump elements. As microparticles are used in the many fields, including cosmetics, biomedical engineering, etc., a relatively simple quantification tool for quantifying them is useful for various purposes including quality control and medical diagnosis.

In one example, microparticles quantification can be used as a method to capture microparticles that are functionalized to detect a certain type of biomarker. In the biomedical field, microparticles are often functionalized to bond specifically and selectively to certain biological target such as oligonucleotides, proteins, or amino acids which is used for detection of diseases and body anomalies. By reacting microparticles with a certain specimen containing the biological target, some biological target will successfully bind to the microparticles, and some will not. If the biological target can simultaneously bind to another immobile entity, such as a flat substrate or a porous material, such binding would result in changes of the quantity of free microparticles in the supernatant. By injecting it into the microfluidic device, it is possible to measure and compare the number of microparticles through the microparticles counter, enabling microparticles quantification and further analysis.

In one example, a form of analysis can be done by combining two distinct types of microparticles with different properties, with proper functionalization of both microparticle to capture a specific target forming a microparticle-target-microparticle assembly. If one of the microparticle has an inherent property which allows for capture such as magnetism, a magnet can be used to capture the magnetic microparticles as well as the microparticle-target-microparticle assembly. The number of the free, non-magnetic microparticles can be counted using some embodiments of the invention as an indirect indicator to measure the concentration level of the target.

As an example, an embodiment of the invention can be applied for rapid tests for COVID-19 antibodies. Some studies have shown that measurement of mucosal antibody level in, for example, nasal mucus may correlate with COVID-19 immune protection and is suitable for self-tests. In an embodiment of the invention, the collected specimen (nasal mucus) may be mixed with magnetic microparticles (MMPs) and polymer microparticles (PMPs) designed to simultaneously bind to mucosal antibody. After magnetic separation that removes MMPs and MMP-antibody-PMPs, the solution of free PMPs can then be loaded to the microfluidic device of the invention, which includes the inlet, the trap, and the capillary pump. As such, the antibody levels can be visualized by the length / amount of PMP accumulation.

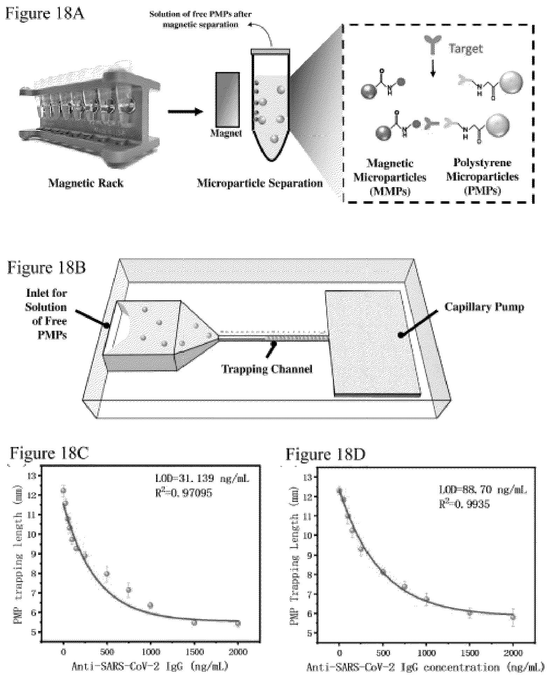

Figure 18A illustrates a schematic design for a COVID-19 mucosal antibody test. which is firstly mixed with microparticles designed to bind to mucosal antibody simultaneously. The reaction solution is then placed in a magnetic rack designed to attract MMPs or PMPs that are bound with MMPs through the presence of COVID-19 mucosal antibodies.

Figure 18B is a schematic diagram of a microfluidic device for detecting an amount of unbound PMPs from Figure 18A. The unbounded PMPs remaining in the solution are then injected into the prototype device shown in Figure 18B which includes a sample inlet, trapping channel, and porous membrane capillary pump.

Figure 18C is a standard curve of different antibody concentrations v. PMP accumulations/trapping length in sensitive mode. Figure 18 C shows the standard curves of different PMP accumulation/trapping length in relation with different concentrations of COVID-19 mucosal antibodies in sensitive mode (1-hour reaction time).

Figure 18D is a standard curve of different antibody concentrations v. PMP accumulations/trapping length in rapid mode. Figure 18D shows the standard curves of different PMP accumulation/trapping length in relation with different concentrations of COVID-19 mucosal antibodies in rapid mode (10-minute reaction time).

Both sensitive and rapid mode give the limit of detection (LOD) of 31.139 and 88.7 ng/mL respectively. Both of these detection limit values are under the antibody concentration protection threshold of 1,441 ng/mL which makes it a viable option for quantitative determination of COVID-19 mucosal antibodies.

Without intending to be limited by theory, it is believed that compared to traditional immunoassays that are either laboratory-based (ELISA) or non-quantitative (LFIA), the detection method and kit herein is expected to provide 1) a better correlation with immune protection, 2) higher sensitivity to reduce false negatives, 3) visual quantification to allow clear-cut results while avoiding ambiguity, and 4) painless sampling of, for example, nasal mucus based on e.g., a nasal swab. Thus, the present invention may allow kits for people to selfmonitor their level of immunity and immunity durability after vaccination. Furthermore, it is believed that this is particularly timely in the post-vaccine era as it alerts the necessity of revaccination when the antibody levels are low, especially when, for example, communities adopt the "living with COVID" strategy. If widely accepted, the measurement result may also serve as antibody-based "immunity passport" for better evaluation before resuming work, flight travel, border entry control, etc. Essentially, such an invention provides rapid pandemic recovery across society and may help to bring normality.

In one example, the microfluidic device can be used as a simple on-site quality checking device. Due to the widespread utilization of microparticles around multiple discipline such cosmetic products and drug delivery systems, being able to perform a quick measurement of particle number can be important because they can readily indicate whether dosage of particular chemicals is present.

Embodiments of the invention can provide various advantage(s), including but not limited to the following. For example, some embodiments of the invention provide a simple microfluidic device which serves as a quick method for an on-site power free measurement of numbers of microparticles by utilizing their geometrical properties. Since the microfluidic device is made portable and does not require an external power capillary pump, it can be readily used or operated in different settings. For example, some embodiments of the invention, particle counting is performed with the assistance of indicator(s) along the fluid channel and an observable length of the stacked microparticles. Thus, the measurement could be done without the need for laboratory specific equipment which may not be readily accessible. For example, in some embodiments, the functionalization of microparticles can be customized to bind to any target. For example, in some embodiments, the microparticles size can be adapted by adjusting the nozzle size or by changing the average pore of the filter paper. For example, in some embodiments, due to the simplicity of the microfluidic device, the microfluidic device can be easily modified with other elements to enhance detection or to do a more complex detection in one single device (i.e., combined with a separator including a magnetic element and a magnetic capture mechanism).

For example, some embodiments of the invention provide a device that can be made easily and/or cheaply. Specifically, in some embodiments, fabrication of the microfluidic device does not use complex protocols such as photolithography and instead uses simple and rapid processes such as additive manufacturing (3D printing) or injection molding. As a result, in different embodiments, the microfluidic device may be made of different materials for different purposes or objectives such as a long shelf life or biocompatibility. Also, with the simplified fabrication process, the microfluidic device in some embodiments of the invention can be produced, or mass produced, more effectively and/or efficiently.

As an example, some embodiments of the invention can be applied for rapid tests for COVID-19 antibodies. Some studies have shown that measurement of mucosal antibody level in nasal mucus may correlate with COVID-19 immune protection and is suitable for self-tests. In some embodiments of the invention, the collected specimen (nasal mucus) may be mixed with magnetic microparticles (MMPs) and polymer microparticles (PMPs) designed to bind to mucosal antibody simultaneously. The sample solution can then be loaded to the microfluidic device of the invention, which includes magnetic separator between the inlet and the trap. As such, after magnetic separation that removes MMPs and MMPs-antibodies-PMPs, the antibody levels can be visualized by the length of PMP accumulation in our invention.

A detection kit based on the microfluidic device of the present invention may provide a better correlate to immune protection, a higher sensitivity to prevent false negatives, a visual quantification to allow clear-cut results without ambiguity, and/or a relatively conformable sampling of nasal mucus based on the nasal swab. The detection kit may be used for selfmonitoring of the level of immunity and its durability after vaccination, which is useful to show when the antibody level goes low and hence when further vaccination might become necessary.

It will be appreciated by persons skilled in the art that numerous variations and/or modifications may be made to the invention as shown in the specific embodiments to provide other embodiments of the invention. The described embodiments of the invention should therefore be considered in all respects as illustrative, not restrictive. Example optional features of some aspects of the invention are set forth in the above summary of the invention. Some embodiments of the invention may include one or more of these optional features (some of which are not specifically illustrated in the drawings). Some embodiments of the invention may lack one or more of these optional features (some of which are not specifically illustrated in the drawings). One or more features in one embodiment and one or more features in another embodiment may be combined to provide further embodiment(s) of the invention. For example, the microfluidic device may be of different form, i.e., not necessarily a microchip, and made in a different scale. For example, the separator, if any, need not be magnetic-based and can perform separation based on chemical or physical or electrical interactions. The shape and form of the channels can be scaled or adjusted accordingly. The number of inlets, channels, outlets, etc., can be more than one. The sequential order of the inlets and processing arrangement can be alternated. The microfluidic device can be used with different types of solution, including but not limited to one with magnetic micro-particles and polymeric micro-particles. The amount of trapped species that is visible may be correlated with an amount of target species in the sample solution. The embodiments are, therefore, to be considered in all respects as illustrative, not restrictive.

## Claims

1. A microfluidic device for facilitating visual determination of a species in a sample solution, the microfluidic device comprising:
an inlet arranged to receive a sample solution; and
a trap in fluid communication with the inlet, wherein the trap comprises a fluid channel, wherein the fluid channel is arranged to substantially trap the species, and wherein the fluid channel is arranged such that the species is visible in the trap;

2. The microfluidic device of claim 1, wherein the microfluidic device further comprises a capillary pump arranged for causing or facilitating movement of the sample solution towards the trap.

3. The microfluidic device of claim 1, wherein the species comprises a plurality of species.

4. The microfluidic device of claim 1, wherein the sample solution comprises a processed solution.

5. The microfluidic device of claim 2,
wherein the capillary pump is disposed downstream of the fluid channel; and
wherein the capillary pump is operable to substantially block passage of the species.

6. The microfluidic device of claim 2, wherein the capillary pump is a removable capillary pump.

7. The microfluidic device of claim 2, wherein the capillary pump is disposed immediately downstream of the fluid channel.

8. The microfluidic device of claim 2, wherein the capillary pump comprises a porous material or a series of microstructures

9. The microfluidic device of claim 8, wherein the porous-material-based capillary pump comprises a filter.

10. The microfluidic device of claim 2, wherein the capillary pump is disposed downstream of the trap.

11. The microfluidic device of claim 8, wherein an average pore size of the porous material is smaller than a diameter of the species.

12. The microfluidic device of claim 1,
wherein the microfluidic device further comprises a processing arrangement for processing the sample solution received at the inlet to provide the processed solution; and
wherein the processing arrangement comprises for separating one or more species from the sample solution received at the inlet to provide a processed solution, the processing arrangement further comprising an arrangement selected from the group of an extractor for facilitating extraction of, or for extracting, the species from the sample solution, a reactor for facilitating reaction of the sample solution with a reactant, a mixer for facilitating mixing of the sample solution with another solution or a substance, and a combination thereof.

13. The microfluidic device of claim 1, wherein the microfluidic device is devoid of a processing arrangement between the inlet and the trap.

14. The microfluidic device of claim 13, wherein the fluid channel further comprises a tapered or narrowed portion for substantially blocking passage of the species, substantially trapping the species.

15. The microfluidic device of claim 2, wherein the trap further comprises:
an indicator arranged along the fluid channel for indicating the presence of the species.

16. The microfluidic device of claim 15, wherein the indicator comprises a reference marking.

17. The microfluidic device of claim 15, wherein the one or more indicators are for indicating a relative or absolute amount of the one or more trapped species.

18. A method for visually detecting or determining a species in a sample solution by the steps of:
a. providing a microfluidic device comprising:
i.an inlet arranged to receive a sample solution; and
ii.a trap in fluid communication with the inlet, wherein the trap comprises a fluid channel, wherein the fluid channel is arranged to substantially trap the species, and wherein the fluid channel is arranged such that the species is visible in the trap;
b. providing a sample solution, the sample solution comprising a species;
c. adding the sample solution to the inlet;
d. collecting the species in the trap; and
e. visually detecting the species in the trap.

19. The method according to claim 18, further comprising the step of processing the sample solution into a processed solution.

20. The method according to claim 19, wherein the processing step occurs within the microfluidic device.
